# EUROPEAN PATENT APPLICATION

(11) **EP 3 858 323 A1**
(43) Date of publication of application: **04.08.2021**
(21) Application number: 19864318.1
(22) Date of filing: 27.09.2019
(51) Int. Cl.: A61K 8/29, A61K 8/06, A61K 8/41, A61K 8/44, A61K 8/55, A61Q 1/02

(54) **WATER-IN-OIL EMULSIFIED SOLID COSMETIC**

(30) Priority: 28.09.2018 JP 2018185892
(71) Applicant: Shiseido Company, Ltd., Chuo-ku Tokyo 104-0061 (JP)
(72) Inventor: TOYAMA, Mai, Tokyo 104-0061 (JP); IKEDA, Tomoko, Tokyo 104-0061 (JP)
(74) Representative: Ter Meer Steinmeister & Partner
(86) International application number: PCT/JP2019/038140
(87) International publication number: WO 2020/067420

(57) **Abstract**

An objective of the present invention is to provide a solid water-in-oil emulsion cosmetic that significantly improves stability at high temperatures, while maintaining an excellent feeling in use (a smooth and watery texture) that is obtained by blending a powder into an internal water phase. The present invention relates to a solid water-in-oil emulsion cosmetic containing an internal water phase in which an internal phase powder, including hydrophilic titanium oxide, is dispersed in an aqueous medium, and an external oil phase that is thickened or solidified with a wax and/or a gelling agent, wherein the solid water-in-oil emulsion cosmetic contains a cationic dispersant and/or an amphoteric dispersant for dispersing the internal phase powder; and the internal phase powder contains 5.0% by mass or more of the hydrophilic titanium oxide relative to the total amount of the cosmetic.

## Description

### TECHNICAL FIELD

The present invention relates to a solid water-in-oil emulsion cosmetic in which a large amount of powder is blended into the internal phase. More specifically, the present invention relates to a solid water-in-oil emulsion cosmetic in which the high-temperature stability and feeling in use (spreadability (lightness), fitting sensation, wateriness, etc.) are improved, while a large amount of a hydrophilic powder containing hydrophilic titanium oxide is blended into the internal water phase.

### BACKGROUND ART

Solid cosmetics of the water-in-oil emulsion type have the characteristics of exceptional water resistance compared to those of the oil-in-water emulsion type, and also allowing emollient oils, oil-soluble medicinal agents, ultraviolet absorption agents and the like to be efficiently blended. However, they have a poor sense of freshness, and there could sometimes be a sticky or oily feeling in use. Additionally, conventional solid water-in-oil emulsion cosmetics, particularly makeup cosmetics, are generally produced by dispersing a hydrophobic powder, such as a pigment that has been subjected to a hydrophobic treatment, in an external oil phase, and solidifying the external oil phase with a solid wax or the like. Thus, the powder particles can become adsorbed to the wax and undergo aggregation, and these aggregates could cause problems such as difficulty in spreading when applied, and reduced wateriness. Furthermore, by subjecting the powders such as pigments to hydrophobic treatments, the oil absorption capacity of the powder was lowered, thereby making it difficult to sufficiently prevent makeup patchiness and makeup deterioration due to sebum.

Therefore, solid water-in-oil emulsion cosmetics in which a powder is dispersed in the internal water phase, thereby providing a lightly spreadable watery texture and improved makeup retention with respect to sebum have been proposed. For example, Patent Document 1 describes a solid water-in-oil emulsion cosmetic in which the external oil phase is solidified with a wax and/or a gelling agent, wherein a cosmetic that is lightly spreadable when applied, that has a watery texture and that has excellent makeup retention is obtained by dispersing a powder in the internal water phase by using a powder dispersant such as a non-ionic surfactant, a fatty acid soap or a condensed phosphate compound. However, in the examples specifically disclosed in Patent Document 1, only one of a wax or a gelling agent is blended therein.

Patent Document 2 describes that, by simultaneously blending a wax with a gelling agent, particularly a specific organically modified clay mineral known as disteardimonium hectorite, an internal phase powder can be stably blended into an internal water phase without using a powder dispersant, thereby improving the high-temperature stability in comparison with Patent Document 1. Additionally, Patent Document 2 suggests that the uniformity of powder dispersion in the internal water phase is improved by blending the same powder dispersants as those in Patent Document 1, specifically, a non-ionic surfactant, a fatty acid soap or a condensed phosphate compound. However, with the formulations disclosed in Patent Documents 1 and 2, the stability at high temperatures, particularly when blending a large amount of powders into the internal water phase, was still insufficient.

### RELATED ART

### PATENT DOCUMENTS

Patent Document 1: JP 2009-137900 A
Patent Document 2: JP 2017-31149 A

### SUMMARY OF THE INVENTION

### PROBLEM TO BE SOLVED BY THE INVENTION

Thus, a problem addressed by the present invention is to provide a solid water-in-oil emulsion cosmetic that further improves stability, particularly at high temperatures, and that allows a large amount of powders to be stably blended into the internal water phase, while maintaining excellent cosmetic retention properties and feeling in use, such as spreadability (lightness), fitting sensation and wateriness.

### MEANS FOR SOLVING THE PROBLEM

As a result of performing diligent research towards solving the above-mentioned problem, the present inventors discovered that, in a solid water-in-oil emulsion cosmetic in which the external oil phase is thickened or solidified with a wax and/or a gelling agent, not only can a large amount of powder, including hydrophilic titanium oxide, be stably blended into the internal water phase, but also, the high-temperature stability and the feeling in use can be further improved by blending, as a powder dispersant, a cationic dispersant and/or an amphoteric dispersant instead of or in addition to a conventional anionic or non-ionic dispersant.

In other words, the present invention provides a solid water-in-oil emulsion cosmetic containing an internal water phase in which an internal phase powder, including hydrophilic titanium oxide, is dispersed in an aqueous medium, and an external oil phase that is thickened or solidified with a wax and/or a gelling agent, wherein the solid water-in-oil emulsion cosmetic contains a cationic dispersant and/or an amphoteric dispersant for dispersing the internal phase powder; and the internal phase powder contains 5.0% by mass or more of the hydrophilic titanium oxide relative to the total amount of the cosmetic.

### EFFECTS OF THE INVENTION

The solid water-in-oil emulsion cosmetic of the present invention has a powder such as titanium oxide blended into the internal water phase, and thus, can be lightly spread when applied to the skin, has a good fitting sensation, and not only provides a watery texture and a powder finish sensation, but also has good oil absorption capacity and excellent makeup retention. Additionally, the solid water-in-oil emulsion cosmetic of the present invention uses a cationic and/or amphoteric dispersant as a powder dispersant for dispersing the hydrophilic powder blended into the internal water phase, and thus has significantly improved stability and can maintain stability for a long time, even under high-temperature conditions. Furthermore, the solid water-in-oil emulsion cosmetic of the present invention also has excellent resistance to drop impacts at high temperatures.

### MODES FOR CARRYING OUT THE INVENTION

The solid water-in-oil emulsion cosmetic of the present invention (hereinafter also referred to as "cosmetic of the present invention") has an external oil phase that is thickened or solidified with a wax and/or a gelling agent, and has a hydrophilic powder (internal phase powder) containing hydrophilic titanium oxide stably dispersed in the internal water phase.

The expression "solid" used in the present specification is interpreted in accordance with the meaning normally used in the cosmetics field, and for example, can be defined as a form or a state in which the composition overall does not exhibit fluidity at temperatures of 50 °C or lower, and in which extreme deformation is not exhibited under normal storage conditions.

Hereinafter, the components constituting the solid water-in-oil emulsion cosmetic of the present invention will be explained in detail.

In the internal water phase of the solid water-in-oil emulsion cosmetic of the present invention, a hydrophilic powder containing hydrophilic titanium oxide is dispersed in the aqueous medium. In the present specification, the hydrophilic powder blended (dispersed) in the internal water phase is referred to as an "internal phase powder".

The "internal phase powder" in the present invention is a powder that can normally be used in a cosmetic product or a pharmaceutical product, and that refers to a surface-hydrophilic powder that can be stably dispersed in an aqueous system. Surface-hydrophilic powders include hydrophilic powders that have not been subjected to a hydrophobic surface treatment, and to hydrophobic or hydrophilic powders that have been surface-treated with a hydrophilic substance.

The internal phase powder in the present invention contains hydrophilic titanium oxide as an essential component. The hydrophilic titanium oxide is not particularly limited, but may be selected from pigment-grade titanium oxide having an average primary particle size of 200 nm or larger (for example, 200 to 400 nm) and/or fine-particle titanium oxide having an average primary particle size of 100 nm or smaller (for example, 10 to 50 nm). Pigment-grade titanium oxide contributes to improving makeup concealment effects, and fine-particle titanium oxide contributes to improving the ultraviolet screening effects of the cosmetic. Since the surface of titanium oxide is inherently hydrophilic, untreated titanium oxide may be used as hydrophilic titanium oxide. On the other hand, titanium oxide or the like having a surface coated with a hydrophilic substance (for example, silica or the like) may also be used.

The internal phase powder in the cosmetic of the present invention contains 5% by mass or more of hydrophilic titanium oxide relative to the total amount of the cosmetic. If the blended amount is less than 5% by mass, then sufficient concealment effects or ultraviolet screening effects cannot be obtained. Although the upper limit of the blended amount is not particurly limited, it should normally be 30% by mass or less. Therefore, the preferable range of the blended amount of the hydrophilic titanium oxide is 5% to 30% by mass, 8% to 25% by mass, 10% to 20% by mass, or the like.

The cosmetic of the present invention preferably contains one or both of pigment-grade titanium oxide and fine-particle titanium oxide. In particular, it is preferable to blend at least hydrophilic fine-particle titanium oxide.

The internal phase powder may contain another hydrophilic powder in addition to the hydrophilic titanium oxide. Specific examples of other hydrophilic powders include inorganic powders such as extender pigments (for example, talc, kaolin, sericite, white mica, synthetic mica, gold mica, red mica, black mica, lithia mica, vermiculite, magnesium carbonate, calcium carbonate, diatomaceous earth, magnesium silicate, calcium silicate, aluminum silicate, barium silicate, strontium silicate, tungstic acid metal salts, silica, hydroxyapatite, zeolite, boron nitride, ceramic powder, etc.); hydrophilic colorants, for example, inorganic white pigments (with the proviso that pigment-grade titanium oxide is excluded; for example, zinc oxide, etc.); inorganic red pigments (for example, iron titanate); inorganic violet pigments (for example, mango violet, cobalt violet, etc.); inorganic green pigments (for example, chromium oxide, chromium hydroxide, cobalt titanate, etc.); inorganic blue pigments (for example, ultramarine blue, Prussian blue, etc.); pearlescent pigments (for example, titanium oxide-coated mica, titanium oxide-coated bismuth oxychloride, titanium oxide-coated talc, colored titanium oxide-coated mica, bismuth oxychloride, argentine, etc.); red iron oxide, yellow iron oxide, black iron oxide, carbon black; metal powder pigments (aluminum powder, copper powder, etc.); organic pigments such as lakes with zirconium, barium, aluminum or the like (for example, Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, Blue No. 404, Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, Blue No. 1, etc.); and natural pigments (for example, chlorophyll, β-carotene, etc.); among which one type or a combination of two or more types may be used.

The blended amount of the internal phase powder (the total blended amount of hydrophilic titanium oxide and other hydrophilic powders) in the solid water-in-oil emulsion cosmetic of the present invention should be 5.0% by mass or more, preferably 5.5% to 30% by mass, and more preferably 6% to 25% by mass relative to the cosmetic. If the blended amount of the powder component is less than 5.0% by mass, then the cosmetic effects due to the powder cannot be sufficiently obtained, and if the blended amount exceeds 30% by mass, then the powder cannot be fully dispersed in the water phase and aggregation occurs, or the powder moves from the water phase into the oil phase, which sometimes causes the feeling µin use to become worse.

In the internal water phase of the solid water-in-oil emulsion cosmetic of the present invention, the above-mentioned internal phase powder is stably and well dispersed in an aqueous medium by a powder dispersant. The "powder dispersant" in the present specification refers to a substance that can be adsorbed to the surface of the powder component, thereby suppressing the aggregation of the powder component and allowing the powder component to be evenly dispersed in the aqueous medium.

One characteristic of the cosmetic of the present invention is that a cationic dispersant and/or an amphoteric dispersant is used as the powder dispersant. A cationic dispersant is a powder dispersant having a cationic group in the molecule, and an amphoteric dispersant is a powder dispersant having both an anionic group and a cationic group in the molecule.

The cationic and amphoteric powder dispersants used in the solid water-in-oil emulsion cosmetic of the present invention may be cationic surfactants and amphoteric surfactants that are normally used in cosmetic products and pharmaceutical products, and are not particularly limited. Examples of cationic powder dispersants include distearyldimonium chloride (product name Cation DSV, manufactured by Sanyo Chemical Industries, Ltd.), behentrimonium chloride (product name Catinal BTC-80, manufactured by Toho Chemical Industry Co., Ltd.), dicocoylethylhydroxyethylmonium methosulfate (product name Dehyquart L80, manufactured by BASF), benzalkonium chloride (product name Catinal MB-50A, manufactured by Toho Chemical Industry Co., Ltd.) and the like. Examples of amphoteric powder dispersants include sodium cocoamphoacetate, cocamidopropyl betaine, lauryl betaine, lecithin, hydrogenated lecithin and the like. In the present invention, it is particularly preferable to use distearyldimonium chloride (cationic powder dispersant).

In the cosmetic of the present invention, another powder dispersant such as, for example, a non-ionic or anionic powder dispersant as described in Patent Documents 1 and 2 may also be blended in addition to the abovementioned cationic powder dispersant and/or amphoteric powder dispersant. Specific examples of other powder dispersants include non-ionic surfactants, fatty acid soaps (higher fatty acid salts), condensed phosphate compounds and the like.

The blended amount of the powder dispersant in the cosmetic of the present invention may be adjusted, as appropriate, in accordance with the type and amount of the powder used and/or the amount of the water phase component, but should be 0.1% to 5% by mass, preferably 0.35% to 2% by mass relative to the total amount of the cosmetic. The blended amount of the cationic dispersant and/or the amphoteric dispersant, which are essential ingredients in the present invention, may vary, as appropriate, within the abovementioned ranges. However, if other powder dispersants are also present, then 50% by mass or more of the total amount of the powder dispersants should preferably consist of the cationic and amphoteric dispersants.

The (internal phase powder)/(cationic and amphoteric powder dispersant) mass ratio in the cosmetic of the present invention should, for example, be within the range from 5 to 50, preferably from 10 to 45. Generally, as the mass ratio of the internal phase powder to the powder dispersant becomes higher, the emulsion system is thought to become more unstable. However, in the present invention, the stability can be maintained, even if the internal phase powder/powder dispersant mass ratio is high, due to the use of a cationic and/or amphoteric powder dispersant. Thus, the cosmetic of the present invention may be formulated so that the (internal phase powder)/(cationic and amphoteric powder dispersant) mass ratio is set to be a high value of 20 or higher, or 25 or higher.

The aqueous medium in which the internal phase powder is dispersed by the above-mentioned powder dispersant may be water, or a mixture of water and an aqueous solvent such as, for example, a lower alcohol.

The blended amount of the aqueous medium in the solid water-in-oil emulsion cosmetic of the present invention is generally 1% to 70% by mass, preferably 3% to 60% by mass, and more preferably 5% to 50% by mass. If the blended amount of the water phase component becomes less than 1% by mass, there may be cases in which a sufficient amount of the powder cannot be dispersed.

The solid water-in-oil emulsion cosmetic of the present invention preferably further contains a water-soluble thickener.

Water-soluble thickeners include vegetable polymers such as xanthan gum, cellulose gum, gum arabic, carrageenan, pectin, agar, quinceseed (marmelo) extract, starch, algecolloid (brown algae extract), mucosaccharides, *Tremellα fuciformis* polysaccharides and succinoglycans; microbial polymers such as dextran and pullulan; animal polymers such as collagen, casein and gelatin; starch-based polymers such as carboxymethyl starch and methylhydroxypropyl starch; alginate polymers such as sodium alginate; vinyl polymers such as carboxyvinyl polymers (carbomers); acrylic polymers such as polyoxyethylene polymers, polyoxyethylene-polyoxypropylene copolymers, sodium polyacrylates and polyacrylamides; and inorganic water-soluble polymers such as bentonite, aluminum-magnesium silicate and laponite.

The blended amount of the water-soluble thickener in the solid water-in-oil emulsion cosmetic of the present invention should preferably be 0.005% to 3% by mass, more preferably 0.05% to 2.8% by mass relative to the total amount of the cosmetic. The blended amount includes all numerical values within the aforementioned ranges and all numerical ranges within the aforementioned ranges. For example, the lower limit value of the range of blended amounts may be 0.005% by mass, preferably 0.01% by mass, more preferably 0.05% by mass, or even more preferably 0.1% by mass, and the upper limit value to be combined with any of these lower limit values may be 3% by mass or preferably 2.8% by mass.

In the internal water phase, other optional components may be further blended in addition to the hydrophilic powder (internal phase powder), the powder dispersant and the aqueous medium, which are essential components, and the water-soluble thickener, which is a preferred optional component. The other optional components are not limited, but may, for example, be a humectant, a stabilizer, various types of water-soluble medicinal agents or the like.

Humectants include, for example, 1,3-butylene glycol, polyethylene glycol, propylene glycol, dipropylene glycol (sometimes abbreviated to DPG), hexylene glycol, glycerin, diglycerin, xylitol, maltitol, maltose, D-mannitol, trehalose, erythritol, propanediol, glucose, polyoxyethylene methyl glucoside and the like.

Stabilizers include chelating agents, metal ion sequestrants, preservatives, antioxidants and the like.

Examples of water-soluble medicinal agents include vitamins such as vitamin A, retinol, retinol palmitate, inositol, pyridoxine hydrochloride, benzyl nicotinate, nicotinic acid amide, DL-α-tocopherol nicotinate, magnesium ascorbyl phosphate, ascorbic acid 2-glucoside, vitamin D2 (ergocalciferol), 2-L ascorbic acid dl-α-tocopherol phosphoric acid diester potassium salt, dl-α-tocopherol, dl-α-tocopherol acetate, pantothenic acid, biotin and the like; anti-inflammatory agents such as allantoin, azulene, glycyrrhetinic acid and the like; whiteners such as arbutin, tranexamic acid, potassium 4-methoxysalicylate; saponifying agents such as zinc oxide and tannic acid; sulfur, lysozyme chloride, pyridoxine hydrochloride, γ-oryzanol and the like.

The external oil phase in the solid water-in-oil emulsion cosmetic according to the present invention is thickened or solidified with a wax and/or a gelling agent.

The wax used in the present invention may be selected from among waxes that are conventionally used in makeup cosmetics.

Specific examples include solid oils/fats such as cacao butter, coconut oil, horse oil, hardened coconut oil, palm oil, beef tallow, mutton tallow, hardened castor oil, and hydrogenated vegetable oils; hydrocarbons such as paraffin waxes (linear hydrocarbons), microcrystalline waxes (branched saturated hydrocarbons), ceresin wax, Japan wax, Fischer-Tropsch waxes and the like; waxes such as beeswax, lanolin, carnauba wax, candelilla wax, rice bran wax (rice wax), spermaceti, jojoba oil, bran wax, montan wax, kapok wax, bayberry wax, shellac wax, sugarcane wax, isopropyl lanolate, hexyl laurate, reduced lanolin, hard lanolin, POE lanolin alcohol ether, POE lanolin alcohol acetate, POE cholesterol ether, polyethylene glycol lanolate and POE hydrogenated lanolin alcohol ether; higher fatty acids such as myristic acid, palmitic acid, stearic acid, behenic acid and hydroxystearic acid; and higher alcohols such as cetyl alcohol, stearyl alcohol, behenyl alcohol, myristyl alcohol and cetostearyl alcohol; among which one type or a combination of two or more types may be used. Among the above, it is preferable to use a paraffin wax (linear hydrocarbon), rice bran wax (rice wax), hydroxystearic acid, hardened castor oil, hydrogenated vegetable oil or the like.

Examples of gelling agents used in the present invention include oil-based gelling agents and the like such as organically modified clay minerals, 12-hydroxystearic acid, starch fatty acid esters, dimethylpolysiloxane polymers and dextrin fatty acid esters, among which one type or a combination of two or more types may be used. Among the above, disteardimonium hectorite, which is an organically modified clay mineral that is sold under the product name "Bentone 38V" or "Bentone 38VCG", can be favorably used.

As the above-mentioned waxes and gelling agents, one type or two or more types of either waxes or gelling agents may be used, or one type or two or more types may be selected from each of waxes and gelling agents, and combined.

The total blended amount of the waxes and the gelling agents is preferably 0.1% to 15% by mass, more preferably 0.5% to 10% by mass, and even more preferably 1% to 7% by mass relative to the total amount of the cosmetic. If the total blended amount of the wax and the gelling agent is less than 0.1% by mass, then the oil phase may not sufficiently solidify, and even if 15% by mass is exceeded, the cosmetic may become too hard, making the feeling in use worse.

The external oil phase of the solid water-in-oil emulsion cosmetic of the present invention includes a liquid oil that has been thickened or solidified by the aforementioned wax and/or gelling agent. The liquid oils in the present invention refer to oils that are liquid at ambient temperature (25 °C), which can normally be used in cosmetic products and pharmaceutical products.

Liquid oils that are used in the present invention include, for example, liquid oils/fats such as avocado oil, camellia oil, macadamia nut oil, mink oil, olive oil, castor oil, jojoba oil, triglycerin and glycerin trioctanoate; hydrocarbons such as liquid paraffin, squalane, paraffin, ceresin and squalene; higher fatty acids such as lauric acid, myristic acid, palmitic acid, stearic acid, behenic acid, oleic acid, isostearic acid, linolic acid and linoleic acid; higher alcohols such as lauryl alcohol, cetyl alcohol, stearyl alcohol, behenyl alcohol, oleyl alcohol, monostearyl glycerol ether, monopalmityl glycerol ether, cholesterol, phytosterol and isostearyl alcohol; ester oils such as isononyl isononanoate, isopropyl myristate, cetyl octanoate, octyldodecyl myristate, butyl stearate, decyl oleate, ethylene glycol dioctanoate, diisostearyl malate, trimethylolpropane trioctanoate, trimethylolpropane triisostearate, pentaerythritol tetraoctanoate, glyceryl trioctanoate, glyceryl triisosteareate, ethyl acetate, butyl acetate and amyl acetate; linear silicone oils such as dimethyl polysiloxane, methylphenyl polysiloxane and methylhydrogen polysiloxane; and cyclic silicone oils such as decamethyl cyclopentasiloxane, dodecamethyl cyclopentasiloxane and octamethyl cyclotetrasiloxane; among which one type or a combination of two or more types may be used. In the present invention, it is preferable to blend, as a main component in the liquid oil, a linear silicone oil such as dimethyl polysiloxane, dodecamethyl pentasiloxane or decamethyl tetrasiloxane, or a cyclic silicone oil such as decamethyl cyclopentasiloxane.

A hydrophobic powder (also referred to as an external phase powder) may also be blended into the external oil phase in the cosmetic of the present invention.

The external phase powder in the present specification refers to powders obtained by subjecting hydrophilic powders that have been indicated as examples of the above-mentioned internal phase powders to a hydrophobic surface treatment by means of a silicone, fluorine, Teflon (registered trademark), a fatty acid, a fatty acid soap, lauroyl lysine or the like, and powders having surfaces that are inherently hydrophobic, such as silicone resin powders. Powder components that have been blended into the external oil phases of conventional solid water-in-oil emulsion cosmetics correspond thereto. In particular, it is preferable to blend, as the external phase powder, a spherical powder, which provides the cosmetic with a good texture.

The blended amount of the external phase powder in the solid water-in-oil emulsion cosmetic of the present invention is not particularly limited, and may, for example, be 40% by mass or less, 35% by mass or less, 30% by mass or less, or 20% by mass or less relative to the total amount of the cosmetic. The solid water-in-oil emulsion cosmetic of the present invention also includes embodiments not containing an external phase powder.

Aside from the above-mentioned liquid oils and external phase powders, it is possible to blend, into the external oil phase, oil-based components such as natural or synthetic solid oils and semi-solid oils (with the proviso that the above-mentioned waxes and gelling agents are excluded), oil-soluble ultraviolet absorption agents, fat-soluble vitamins and fragrances.

Ultraviolet absorption agents include benzoic acid-based ultraviolet absorption agents such as para-aminobenzoic acid; anthranilic acid-based ultraviolet absorption agents such as methyl anthranilate; salicylic acid-based ultraviolet absorption agents such as octyl salicylate and phenyl salicylate; cinnamic acid-based ultraviolet absorption agents such as ethylhexyl methoxycinnamate, isopropyl paramethoxycinnamate, octyl paramethoxycinnamate and glyceryl mono-2-ethylhexanoate diparamethoxycinnamate; benzophenone-based ultraviolet absorption agents such as 2,4-dihydroxybenzophenone, 2-hydroxy-4-methoxybenzophenone and 2-hydroxy-4-methoxybenzophenone-5-sulfonic acid; and 2-(2'-hydroxy-5'-methylphenyl)benzotriazole and 4-tert-butyl-4'-methoxybenzoylmethane.

Additionally, in the present invention, an emulsifier that is used when emulsifying the above-mentioned internal water phase int the external oil phase may be blended. Examples of preferable emulsifiers include emulsifiers that can generally be blended into water-in-oil emulsion compositions, i.e., emulsifiers that are lipophilic (HLB = 7 or lower). Specific examples include sorbitan fatty acid esters such as sorbitan monolaurate, sorbitan mono-oleate, sorbitan monoisostearate, sorbitan tristearate and sobitan sequiisostearate; glycerol fatty acid esters such as glycerol monostearate, glycerol mono-oleate, glycerol isostearate, diglycerol diisostearate and decaglyceryl pentaisostearate; polyoxyethylene hardened castor oils such as POE(5), POE(7.5) and POE(10) hardened castor oils; dipolyhydroxystearic acid esters; high-molecular-weight lipophilic activators such as polyglyceryl-2 dipolyhydroxystearate (manufactured by Cognis, PGPH) and PEG30 dipolyhydroxystearate (manufactured by Uniquema, Arlacel P135); polyether-based silicones such as cetyl dimethicone copolyol (for example, "ABIL EM90" (manufactured by Goldschmidt Corp.)), polyether-modified silicones (for example, PEG-10 dimethicone, "KF6017" (manufactured by Shin-etsu Chemical Co., Ltd.)) and crosslinked polyether-modified silicones (for example, the "KSG" series (manufactured by Shin-Etsu Chemical Co., Ltd.)); and polyglycerin-based silicones such as polyglycerin-modified silicones and alkyl-comodified polyglycerin-modified silicones. In the present invention, it is possible to use one type or two or more types of emulsifiers having an HLB of 7 or lower. In the present invention, it is preferable to use a silicone-based emulsifier such as a polyether-modified silicone.

In the solid water-in-oil emulsion cosmetic of the present invention, aside from the above-mentioned components, other optional components that can be blended into cosmetics may be blended into the internal water phase or the external oil phase within a range not compromising the effects of the present invention.

The cosmetic of the present invention can be produced in accordance with a method that is normally used for solid water-in-oil emulsion cosmetics. To explain briefly, it can be produced substantially be the following procedure.
(1) The aqueous medium, the cationic and/or amphoteric powder dispersant, the water-soluble thickener and other optional components are mixed while heating as needed, after which the internal phase powder is added and stirred with a homomixer or the like to prepare the water phase components.
(2) The wax and/or gelling agent, liquid oil and other optional components are mixed while heating as needed to prepare the oil phase components.
(3) An emulsifier is optionally added to the oil phase components, the water phase components are added, and the components are stirred with a homomixer to form an emulsion.
(4) A container is filled with the emulsion and cooled to solidify, thereby obtaining the solid water-in-oil emulsion cosmetic of the present invention.

The solid water-in-oil emulsion cosmetic of the present invention is appropriate for being provided as a makeup cosmetic such as a foundation, an eyeshadow, an eyeliner, a mascara, a rouge or the like, or as a makeup base or a sunscreen. In particular, it can appropriately be formed into a solid foundation with exceptional concealing capacity (covering capacity) and/or ultraviolet screening capacity by blending a large amount of titanium oxide into the internal water phase. In terms of the form, it may be provided as a stick or as a compact. Additionally, the solid water-in-oil emulsion cosmetic of the present invention can also be used as a cushion-type foundation by impregnating a water-insoluble sponge, a foam support or the like therewith.

### EXAMPLES

Although the present invention will be explained in further detail by providing examples below, the present invention is not limited in any way thereby. Where not otherwise noted, the blended amounts represent the percentages by mass relative to the total amount of the cosmetic.

Solid water-in-oil emulsion cosmetics were prepared with the formulations indicated in Table 1 to Table 3 indicated below. Next, for the solid water-in-oil emulsion cosmetic of each example, (1) the high-temperature stability, (2) the drop impact resistance at high temperatures, and (3) the "spreadability (lightness)" the "fitting sensation", the "wateriness" and the "powder finish sensation" were evaluated in the manners indicated below.

Evaluation Methods and Evaluation Criteria
(1) High-temperature stability
   A sample of each example was stored at 80 °C for 2 hours, after which each sample was visually observed and evaluated under the criteria indicated below.
   A: The sample (emulsion) was stable and absolutely no changes in appearance were observed.
   B: The sample surface was uniform and slight separation (or aggregation) was observed in the interior, but of a level presenting no problems in use.
   C: Separation was observed in the entire sample.
(2) Drop impact resistance at high temperatures
   A sample of each example was adjusted to 45 °C and dropped from a height of 30 cm, after which each sample was visually observed and evaluated under the criteria indicated below.
   A: Absolutely no changes were observed in the appearance of the sample.
   B: Some cracking (or lift-up from the container) was observed in the sample, but of a level presenting no problems in use.
   C: The external shape of the sample was not able to be maintained.
(3) Actual usage test (spreadability, fitting sensation, wateriness and powder finish sensation)
   A sample of each example was applied to the cheeks of 20 expert panelists and evaluated under the criteria indicated below.
   A: 18 or more out of 20 replied that the sample had good spreadability, a fitting sensation, wateriness or a powder finish sensation.
   B: 8 to 17 out of 20 replied that the sample had good spreadability, a fitting sensation, wateriness or a powder finish sensation.
   C: 7 or fewer out of 20 replied that the sample had good spreadability, a fitting sensation, wateriness or a powder finish sensation.
      -: Could not be evaluated.
      Note that "powder finish sensation" refers to a texture that is moist during application, but that, after application, is dry, as if a powdery foundation has been applied.

**[Table 1]**

| | | Ex. 1 | Comp. Ex. 1 | Comp. Ex. 2 | Comp. Ex. 3 | Ex. 2 | Ex. 3 |
|---|---|---|---|---|---|---|---|
| Water | Water | bal | bal | bal | bal | bal | bal |
| Stabilizer | Chelating agent | s.a. | s.a. | s.a. | s.a. | s.a. | s.a. |
| | Metal ion sequestrant | s.a. | s.a. | s.a. | s.a. | s.a. | s.a. |
| | Preservative | 0.35 | 0.35 | 0.35 | 0.35 | 0.35 | 0.35 |
| | Antioxidant | 0.02 | 0.02 | 0.02 | 0.02 | 0.02 | 0.02 |
| Water-soluble Thickener | Xanthan gum | 0.10 | - | 0.10 | 0.10 | 0.10 | 0.10 |
| | Cellulose gum | 0.05 | - | 0.05 | 0.05 | 0.05 | 0.05 |
| Humectant | Glycerin | 5.00 | 5.00 | 5.00 | 5.00 | 5.00 | 5.00 |
| | DPG | 5.00 | 5.00 | 5.00 | 5.00 | 5.00 | 5.00 |
| Internal Phase Powder | Silica-coated iron oxide (yellow) | 1.25 | 1.25 | 1.25 | 1.25 | 4.50 | 1.25 |
| | Silica-coated iron oxide (red) | 0.30 | 0.30 | 0.30 | 0.30 | 1.30 | 0.30 |
| | Silica-coated iron oxide (black) | 0.10 | 0.10 | 0.10 | 0.10 | 0.10 | 0.10 |
| | Silica-coated pigment-grade titanium oxide | 8.00 | 8.00 | 2.00 | 8.00 | 8.00 | 8.00 |
| | Silica-coated fine-particle titanium oxide | 5.00 | 5.00 | 3.00 | 5.00 | 7.00 | 5.00 |
| Dispersant | Distearyldimonium chloride | 0.50 | - | - | - | 0.80 | 0.50 |
| | Palmitic acid | 0.30 | - | - | 0.30 | 0.50 | 0.30 |
| Liquid Oil | Dodecamethyl pentasiloxane | 30.00 | 30.00 | 30.00 | 30.00 | 30.00 | 30.00 |
| | Dimethyl polysiloxane | 2.50 | 2.50 | 2.50 | 2.50 | 2.50 | 2.50 |
| | Ethylhexyl methoxycinnamate | 3.50 | 3.50 | 3.50 | 3.50 | 3.50 | 3.50 |
| Activator | Sorbitan sesquiisostearate | 0.70 | 0.70 | 0.70 | 0.70 | 0.70 | 0.70 |
| | PEG-10 dimethicone | 2.00 | 2.00 | 2.00 | 2.00 | 2.00 | 2.00 |
| Gelling Agent | Disteardimonium hectorite | 0.25 | 0.25 | 0.25 | 0.25 | 0.25 | 0.25 |
| External Phase Powder | Silica dimethyl silylate | 0.30 | 0.30 | 0.30 | 0.30 | 0.30 | 0.30 |
| | Polymethyl silsesquioxane | 7.50 | 7.50 | 3.00 | 7.50 | 7.50 | 10.00 |
| | Barium sulfate | 5.50 | 5.50 | 5.50 | 5.50 | 5.50 | 8.00 |
| Wax | Paraffin wax | 3.00 | 3.00 | 3.00 | 3.00 | 3.00 | 3.00 |
| | Rice bran wax | 1.00 | 1.00 | 1.00 | 1.00 | 1.00 | 1.00 |
| | Total | 100.00 | 100.00 | 100.00 | 100.00 | 100.00 | 100.00 |
| | Stability | A | c | B | c | A | A |
| | Drop impact resistance | A | - | C | - | A | A |
| | Spreadability | A | - | A | - | A | A |
| | Fitting sensation | A | - | B | - | A | A |
| | Wateriness | A | - | A | - | A | A |
| | Powder finish sensation | A | - | B | - | A | A |

**[Table 2]**

| | | Ex. 4 | Ex. 5 | Ex. 6 | Ex. 7 | Ex. 8 | Ex. 9 | Ex. 10 |
|---|---|---|---|---|---|---|---|---|
| Water | Water | bal | bal | bal | bal | bal | bal | bal |
| Stabilizer | Chelating agent | s.a. | s.a. | s.a. | s.a. | s.a. | s.a. | s.a. |
| | Metal ion sequestrant | s.a. | s.a. | s.a. | s.a. | s.a. | s.a. | s.a. |
| | Preservative | 0.35 | 0.35 | 0.35 | 0.35 | 0.35 | 0.35 | 0.35 |
| | Antioxidant | 0.02 | 0.02 | 0.02 | 0.02 | 0.02 | 0.02 | 0.02 |
| Water-soluble Thirkener | Xanthan gum | 0.10 | 0.10 | - | - | 0.10 | 0.10 | 0.10 |
| | Cellulose gum | 0.05 | 0.05 | 0.03 | 0.03 | 0.05 | 0.05 | 0.05 |
| | Succinoglycan | - | - | 0.50 | 0.10 | - | - | - |
| Humectant | Glycerin | 5.00 | 5.00 | 5.00 | 5.00 | 5.00 | 5.00 | 5.00 |
| | DPG | 5.00 | 5.00 | 5.00 | 5.00 | 5.00 | 5.00 | 5.00 |
| Internal Phase Powder | Silica-coated iron oxide (yellow) | 1.25 | 1.25 | 1.25 | 1.25 | 1.25 | 1.25 | 1.25 |
| | Silica-coated iron oxide (red) | 0.30 | 0.30 | 0.30 | 0.30 | 0.30 | 0.30 | 0.30 |
| | Silica-coated iron oxide (black) | 0.10 | 0.10 | 0.10 | 0.10 | 0.10 | 0.10 | 0.10 |
| | Silica-coated pigment-grade titanium oxide | 8.00 | 8.00 | 8.00 | 8.00 | 8.00 | 8.00 | 8.00 |
| | Silica-coated fine-particle titanium oxide | 5.00 | 5.00 | 5.00 | 5.00 | 5.00 | 5.00 | 5.00 |
| Dispersant | Distearyldimonium chloride | 0.50 | 0.50 | 0.50 | 0.50 | 0.50 | 0.50 | 0.50 |
| | Palmitic acid | 0.30 | 0.30 | 0.30 | 0.30 | 0.30 | 0.30 | 0.30 |
| Liquid Oil | Dodecamethyl pentasiloxane | 30.00 | 30.00 | 30.00 | - | - | 30.00 | - |
| | Dimethyl polysiloxane | 2.50 | 2.50 | 2.50 | 2.50 | 2.50 | 2.50 | - |
| | Decamethyl tetrasiloxane | - | - | - | 30.00 | - | - | 30.00 |
| | Decamethyl cyclopentasiloxane | - | - | - | - | 20.00 | - | 2.50 |
| | Isododecane | - | - | - | - | 5.00 | - | - |
| | Ethylhexyl methoxycinnamate | 3.50 | 3.50 | 3.50 | 3.50 | 8.50 | 3.50 | - |
| Activator | Sorbitan sesquiisostearate | 0.70 | 0.70 | 0.70 | 0.70 | 0.70 | 0.70 | - |
| | PEG-10 dimethicone | 2.00 | 2.00 | 2.00 | 2.00 | 2.00 | 2.00 | 3.50 |
| Gelling Agent | Disteardimonium hectorite | 0.25 | 0.25 | 0.25 | 0.25 | 0.25 | 0.50 | 0.70 |
| | Dimethicone/vinyl dimethicone crosspolymer | - | - | - | - | - | 3.00 | 2.00 |
| | Crosslinked polyether-modified silicone | - | - | - | - | - | - | 0.50 |
| External Phase Powder | Silica dimethyl silylate | 0.30 | 0.30 | 0.30 | 0.30 | 0.30 | 0.30 | - |
| | Polymethyl silsesquioxane | 10.00 | 10.00 | 10.00 | 10.00 | 3.00 | 7.50 | 3.00 |
| | Barium sulfate | 5.00 | - | - | - | 5.50 | 5.50 | 0.30 |
| | Nylon-12 | 3.00 | 3.00 | 5.00 | 5.00 | - | - | 7.50 |
| | (Dimethicone/vinyl dimethicone/methicone) crosspolymer | - | 5.00 | 3.00 | 3.00 | - | - | 5.50 |
| | Zinc oxide | - | - | - | - | - | 1.00 | - |
| | Titanium oxide | - | - | - | - | - | - | 3.00 |
| | Titanated mica | - | - | - | - | - | 3.00 | - |
| Wax | Paraffin wax | 3.00 | 3.00 | 3.00 | 3.00 | 3.00 | 3.00 | 1.00 |
| | Rice bran wax | 1.00 | 1.00 | 1.00 | 1.00 | 1.00 | 1.00 | - |
| | Carnauba wax | - | - | - | - | - | - | 3.00 |
| | Candelilla wax | - | - | - | - | - | - | 1.00 |
| | Total | 100.00 | 100.00 | 100.00 | 100.00 | 100.00 | 100.00 | 100.00 |
| | Stability | A | A | A | A | A | A | A |
| | Drop impact resistance | A | A | A | A | A | A | A |
| | Spreadability | A | A | A | A | A | B | B |
| | Fitting sensation | A | A | B | A | A | A | A |
| | Wateriness | A | A | A | A | A | A | A |
| | Powder finish sensation | A | A | B | A | A | A | A |

**[Table 3]**

| | | Ex. 11 | Ex. 12 | Ex. 13 | Ex. 14 | Ex. 15 | Ex. 16 |
|---|---|---|---|---|---|---|---|
| Water | Water | bal | bal | bal | bal | bal | bal |
| Stabilizer | Chelating agent | s.a. | s.a. | s.a. | s.a. | s.a. | s.a. |
| | Metal ion sequestrant | s.a. | s.a. | s.a. | s.a. | s.a. | s.a. |
| | Preservative | 0.35 | 0.35 | 0.35 | 0.35 | 0.35 | 0.35 |
| | Antioxidant | 0.02 | 0.02 | 0.02 | 0.02 | 0.02 | 0.02 |
| Water-soluble Thickener | Xanthan gum | 0.10 | 0.10 | 0.10 | 0.10 | 0.10 | 0.10 |
| | Cellulose gum | 0.05 | 0.05 | 0.05 | 0.05 | 0.05 | 0.05 |
| Humectant | Glycerin | 5.00 | 5.00 | 5.00 | 5.00 | 5.00 | 5.00 |
| | DPG | 5.00 | 5.00 | 5.00 | 5.00 | 5.00 | 5.00 |
| Internal Phase Powder | Silica-coated iron oxide (yellow) | 1.25 | 1.25 | 1.25 | 1.25 | 1.25 | 1.25 |
| | Silica-coated iron oxide (red) | 0.30 | 0.30 | 0.30 | 0.30 | 0.30 | 0.30 |
| | Silica-coated iron oxide (black) | 0.10 | 0.10 | 0.10 | 0.10 | 0.10 | 0.10 |
| | Silica-coated pigment-grade titanium oxide | 8.00 | 8.00 | 8.00 | 8.00 | 8.00 | 8.00 |
| | Silica-coated fine-particle titanium oxide | - | - | 5.00 | 5.00 | 5.00 | 5.00 |
| | Silicic anhydride-coated zinc oxide | 5.00 | - | - | - | - | - |
| | Low-temperature annealed zinc oxide | - | 5.00 | - | - | - | - |
| Dispersant | Distearyldimonium chloride | 0.50 | 0.50 | 0.50 | 0.50 | - | - |
| | Palmitic acid | 0.30 | 0.30 | 0.30 | 0.30 | 0.15 | 0.20 |
| | Lauryl betaine | - | - | - | - | 0.80 | - |
| | Hydrogenated lecithin | - | - | - | - | - | 0.70 |
| Liquid Oil | Dodecamethyl pentasiloxane | 30.00 | 30.00 | 30.00 | 30.00 | 30.00 | 30.00 |
| | Dimethyl polysiloxane | 2.50 | 2.50 | 2.50 | 2.50 | 2.50 | 2.50 |
| | Ethylhexyl methoxycinnamate | 3.50 | 3.50 | 3.50 | 3.50 | 3.50 | 3.50 |
| Activator | Sorbitan sesquiisostearate | 0.70 | 0.70 | 0.70 | 0.70 | 0.70 | 0.70 |
| | PEG-10 dimethicone | 2.00 | 2.00 | 2.00 | 2.00 | 2.00 | 2.00 |
| Gelling Agent | Disteardimonium hectorite | 0.25 | 0.25 | 0.25 | 0.25 | 0.25 | 0.25 |
| External Phase Powder | Silica dimethyl silylate | 0.30 | 0.30 | 0.30 | 0.30 | 0.30 | 0.30 |
| | Polymethyl silsesquioxane | 7.50 | 7.50 | 7.50 | 7.50 | 7.50 | 7.50 |
| | Barium sulfate | 5.50 | 5.50 | 5.50 | 5.50 | 5.50 | 5.50 |
| | Methyl methacrylate crosspolymer | 3.00 | 3.00 | 3.00 | - | - | - |
| Wax | Paraffin wax | 3.00 | 3.00 | 3.00 | 3.00 | 3.00 | 3.00 |
| | Rice bran wax | 1.00 | 1.00 | 1.00 | - | 1.00 | 1.00 |
| | Carnauba wax | - | - | - | 0.50 | - | - |
| | Candelilla wax | - | - | - | 0.50 | - | - |
| | Microcrystalline wax | - | - | 1.00 | - | - | - |
| | Total | 100.00 | 100.00 | 100.00 | 100.00 | 100.00 | 100.00 |
| | Stability | A | A | A | A | B | B |
| | Drop impact resistance | A | A | A | A | B | B |
| | Spreadability | A | A | A | A | A | A |
| | Fitting sensation | A | A | A | A | A | A |
| | Wateriness | A | A | A | A | A | A |
| | Powder finish sensation | A | A | A | A | A | A |

As is clear from the results indicated in Tables 1 to 3 above, Examples 1 to 16, which are solid water-in-oil emulsion cosmetics having an internal water phase in which an internal phase powder containing 5% by mass or more of hydrophilic titanium oxide is dispersed in an aqueous medium by a powder dispersant containing a cationic or amphoteric dispersant, and an external oil phase thickened or solidified with a wax and/or a gelling agent, exhibited good results in all of high-temperature stability, spreadability, fitting sensation, wateriness and powder finish sensation. However, Comparative Example 1, which did not contain a powder dispersant and a water-soluble thickener, had significantly worse stability. On the other hand, in the case in which the amount of hydrophilic titanium oxide blended into the internal water phase was relatively low, at 5% by mass, the stability was able to be improved even without blending a dispersant, by blending a water-soluble thickener into the internal water phase. However, the drop impact resistance was insufficient (Comparative Example 2). Additionally, in the case in which the amount of the hydrophilic titanium oxide was increased, stability was not able to be maintained for a long period of time, even when using an anionic powder dispersant (palmitic acid) that is conventionally known (Comparative Example 3).

Other formulation examples of the solid water-in-oil emulsion cosmetic according to the present invention are indicated below. However, the present invention is not limited thereto.

### Formulation Example 1: Solid emulsion foundation

| (Formulation) | (% by mass) |
|---|---|
| (1) Dimethyl polysiloxane | 20 |
| (2) Decamethyl tetrasiloxane | 10 |
| (3) Microcrystalline wax | 3 |
| (4) Ceresin wax | 5 |
| (5) Nylon-12 | 3 |
| (6) Methyl polymethacrylate | 5 |
| (7) Ethylhexyl methoxycinnamate | 3 |
| (8) Cetyl ethylhexanoate | 3 |
| (9) PEG-10 dimethicone | 2 |
| (10) Disteardimonium hectorite | 0.5 |
| (11) Internal phase powder lake | 20 |
| (12) Ion-exchanged water | bal |
| (13) 1,3-Butylene glycol | 3 |
| (14) Diglycerin | 1 |
| (15) Trisodium phosphate | 0.3 |
| (16) Preservative | 0.1 |
| (17) Palmitic acid | 0.5 |
| (18) Behentrimonium chloride | 0.8 |
| (19) Hydroxyethyl cellulose | 0.3 |
| (20) Fragrance | 0.1 |

### (Production method)

(i) After heating and mixing the water phase components (12) to (19), the internal phase powder (11) was added, and this mixture was stirred with a homomixer to obtain a powder dispersion (water phase).
(ii) Next, the aforementioned dispersion (water phase) was added, optionally with an emulsifier, to an appropriately heated and melted mixture of components (1) to (10) and (20), and stirred with a homomixer or the like to form an emulsion.
(iii) Finally, an appropriate container was filled with the aforementioned emulsion, then cooled and solidified, thereby obtaining the solid emulsion foundation of the present formulation example.

The internal phase powder lake was a mixture of 2.8 parts of talc, 18 parts of titanium oxide, 2.36 parts of yellow iron oxide, 0.8 parts of red iron oxide and 0.04 parts of black iron oxide.

### Formulation Example 2: Emulsion blush

| (Formulation) | (% by mass) |
|---|---|
| (1) Isododecane | 6 |
| (2) Glyceryl tri-2-ethylhexanoate | 5 |
| (3) Dodecamethyl pentasiloxane | 15 |
| (4) Microcrystalline wax | 1 |
| (5) Hydrogenated jojoba oil | 3 |
| (6) Candelilla wax | 1 |
| (7) Sorbitan monoisostearate | 1 |
| (8) (Vinyl dimethicone/methicone silsesquioxane) crosspolymer | 2 |
| (9) PEG-10 dimethicone | 2 |
| (10) Disteardimonium hectorite | 0.8 |
| (11) Titanated mica | 5 |
| (12) Internal phase powder lake | 12 |
| (13) Ion-exchanged water | bal |
| (14) Propylene glycol | 6 |
| (15) Polyoxyethylene glycerin monoisostearate | 0.5 |
| (16) Benzalkonium chloride | 1.5 |
| (17) Polyvinyl alcohol | 0.5 |
| (18) Preservative | 0.1 |
| (19) Fragrance | 0.1 |

### (Production method)

After heating and mixing components (13) to (18), component (12) was added and dispersed with a homomixer. Next, a mixture of components (1) to (11) and (19) preheated to 80 °C was added, and emulsified and dispersed. Thereafter, an appropriate container was filled with the mixture while in a state of fluidity, then the mixture was cooled to room temperature to obtain the emulsion blush of the present formulation example.

The internal phase powder lake was a mixture of 1.5 parts of talc, 8.4 parts of titanium oxide and 2.1 parts of red iron oxide.

### Formulation Example 3: Solid emulsion sunscreen

### (Production method)

Components (13) to (19) were heated and mixed at 80 °C, and components (10) to (12) were added, and heated and dispersed with a homomixer. Next, a mixture of components (1) to (9) and (20) preheated to 80 °C was added, and emulsified and dispersed. Thereafter, an appropriate container was filled with the mixture while in a state of fluidity, then the mixture was cooled to room temperature to obtain the objective cosmetic.

### Formulation Example 4: Solid emulsion foundation (impregnated type)

| (Formulation) | (% by mass) |
|---|---|
| (1) Dimethyl polysiloxane | 8 |
| (2) Dimethicone | 20 |
| (3) Microcrystalline wax | 4 |
| (4) Paraffin wax | 3 |
| (5) Diisopropyl sebacate | 4 |
| (6) Ethylhexyl methoxycinnamate | 5 |
| (7) Diethylaminohydroxybenzoyl hexyl benzoate | 1 |
| (8) PEG-10 dimethicone | 2 |
| (9) Disteardimonium hectorite | 0.3 |
| (10) Silica-coated titanium oxide | 3 |
| (11) Silica-coated fine-particle titanium oxide | 6 |
| (12) Silica-coated zinc oxide | 3 |
| (13) Ion-exchanged water | bal |
| (14) Propanediol | 5 |
| (15) Sodium hexametaphosphate | 0.1 |
| (16) Bentonite | 1 |
| (17) Preservative | 0.1 |
| (18) Palmitic acid | 0.2 |
| (19) Distearyldimonium chloride | 0.6 |
| (20) Fragrance | 0.1 |

| (Formulation) | (% by mass) |
|---|---|
| (1) Dodecamethyl pentasiloxane | 25 |
| (2) Glyceryl tri-2-ethylhexanoate | 3 |
| (3) Isododecane | 2 |
| (4) Ethylhexyl methoxycinnamate | 5 |
| (5) Polyglyceryl diisostearate | 0.5 |
| (6) PEG-10 dimethicone | 3 |
| (7) Disteardimonium hectorite | 0.5 |
| (8) Calcium carbonate | 10 |
| (9) Paraffin wax | 0.5 |
| (10) Beeswax | 1 |
| (11) Internal phase powder lake | 15 |
| (12) Ion-exchanged water | bal |
| (13) Disodium edetate | 0.2 |
| (14) Sodium hexametaphosphate | 0.3 |
| (15) Preservative | 0.5 |
| (16) Quinceseed extract | 0.2 |
| (17) Glycerin | 3 |
| (18) Dipropylene glycol | 3 |
| (19) Distearyldimonium chloride | 0.4 |
| (20) Palmitic acid | 0.3 |

### (Production method)

Components (12) to (20) were heated and mixed at 80 °C, and component (11) was added, then heated and dispersed with a homomixer or the like. Next, a mixture of components (1) to (10) preheated to 80 °C was added, then emulsified and dispersed. Thereafter, an appropriate container was filled with the mixture while in a state of fluidity, then the mixture was cooled to room temperature to obtain the objective cosmetic.

The internal phase powder lake was a mixture of 1.8 parts of talc, 11.25 parts of titanium oxide, 1.4 parts of yellow iron oxide, 0.5 parts of red iron oxide and 0.05 parts of black iron oxide.

## Claims

1. A solid water-in-oil emulsion cosmetic containing an internal water phase in which an internal phase powder, including hydrophilic titanium oxide, is dispersed in an aqueous medium, and an external oil phase that is thickened or solidified with a wax and/or a gelling agent, wherein:
the solid water-in-oil emulsion cosmetic contains a cationic dispersant and/or an amphoteric dispersant for dispersing the internal phase powder; and
the internal phase powder contains 5.0% by mass or more of the hydrophilic titanium oxide relative to the total amount of the cosmetic.

2. The solid water-in-oil emulsion cosmetic according to claim 1, wherein the hydrophilic titanium oxide includes fine-particle titanium oxide.

3. The solid water-in-oil emulsion cosmetic according to claim 1 or 2, wherein the internal phase powder further includes a hydrophilic colorant.

4. The solid water-in-oil emulsion cosmetic according to any one of claims 1 to 3, wherein the blended amount of the internal phase powder is within the range from 5% to 30% by mass relative to the total amount of the cosmetic.

5. The solid water-in-oil emulsion cosmetic according to any one of claims 1 to 4, wherein the blended amount of the cationic dispersant and/or the amphoteric dispersant is 0.1% to 5% by mass with respect to the total amount of the cosmetic.

6. The solid water-in-oil emulsion cosmetic according to any one of claims 1 to 5, wherein the cationic dispersant and/or the amphoteric dispersant is one or more selected from the group consisting of distearyldimonium chloride, behentrimonium chloride, dicocoylethylhydroxyethylmonium methosulfate, benzalkonium chloride, sodium cocoamphoacetate, cocamidopropyl betaine, lauryl betaine, lecithin and hydrogenated lecithin.

7. The solid water-in-oil emulsion cosmetic according to any one of claims 1 to 6, wherein the mass ratio (internal phase powder/dispersant) of the blended amount of the internal phase powder to the total blended amount of the cationic dispersant and the amphoteric dispersant is within the range from 10 to 45.

8. The solid water-in-oil emulsion cosmetic according to any one of claims 1 to 7, further including a water-soluble thickener.

9. The solid water-in-oil emulsion cosmetic according to claim 8, wherein the blended amount of the water-soluble thickener is 0.005% to 3% by mass relative to the total amount of the cosmetic.

10. The solid water-in-oil emulsion cosmetic according to any one of claims 1 to 9, further containing, in the external oil phase, an external phase powder having a hydrophobic surface.

11. The solid water-in-oil emulsion cosmetic according to claim 10, wherein the blended amount of the external phase powder is 40% by mass or lower relative to the total amount of the cosmetic.
